# EUROPEAN PATENT APPLICATION

(11) **EP 1 136 438 A2**
(43) Date of publication of application: **26.09.2001**
(21) Application number: 01301903.9
(22) Date of filing: 01.03.2001
(51) Int. Cl.: B68B 1/04, B68B 5/00

(54) **Measuring apparatus for horse tack**

(30) Priority: 16.03.2000 GB 0006229
(71) Applicant: H.W. Dabbs (Bridlemakers) Ltd., Walsall, West Midlands, WS2 9LN (GB)
(72) Inventor: Dabbs, Harry William, H.W.Dabbs(Bridlemakers) Ltd., Walsall, West Midlands WS2 9LN (GB)
(74) Representative: Watkins, Rosalind Philippa

(57) **Abstract**

Measuring apparatus in the form of a fitting bridle (10) for use in making equine tack includes a strap of adjustable length, the strap being made up of a first strap member and a second strap member joined by connection means (34, 44) whereby the position of the first or second strap member within the connection means (34, 44) may be adjusted to thereby adjust the length of the strap. The strap may form a cheek piece, brow band, throat lash or part of a nose band of the fitting bridle.

## Description

The invention relates to measuring apparatus for use in making tack for horses and ponies and to a method of manufacturing tack for horses and ponies.

Bridles and other tack for horses or ponies often do not fit the animal particularly well. For instance, a bridle for a horse may not sit correctly on the bones in the horse's face and the buckles on the bridle may rub against the places where these bones project. It is particularly difficult to find a nose band that fits a horse correctly. The result is that horse owners may be forced to purchase a standard bridle but then to replace items with parts of the bridle of different sizes. This is expensive and inconvenient.

There is therefore a demand for a made to measure bridle or similar item of horse tack. However, it is not generally commercially feasible for a bridle manufacturer to visit and measure a horse before manufacturing a suitable bridle.

According to the invention there is provided measuring apparatus for use in making equine tack, the measuring apparatus including a strap of adjustable length, the strap being made up of a first strap member and a second strap member joined by connection means, whereby the position of the first strap member within the connection means may be adjusted to thereby adjust the length of the strap, and wherein the connection means is alterable between a state in which it allows the first strap member to slide therethrough and a state in which such sliding is not possible.

Preferably the second strap member passes through the connection means and partially overlaps itself such that the length of the strap may be adjusted by varying the extent of this overlap. Preferably the extent of overlap is continuously variable.

Preferably the overlapped portion of the second strap member may be attached to the remainder of the second strap member by a hook and fleece fastening. Preferably an end portion of the second strap member is provided with hooks and the remainder of the strap member with fleece. The end portion of the strap member may be between 20mm and 60mm long.

Preferably the position of the first strap member within the connection means is adjustable. The first strap member may be provided with one or more indicia for indicating a preferred position or positions within the connection means.

When the connection means is in its open state, the second strap member may also be able to slide therethrough. Alternatively, the connection means may independently allow or prevent the first and second members respectively from sliding therethrough.

The connection means may include a clip member provided with a pivotal tongue. The tongue may be pivotable between an open position in which the first and/or second strap member may pass through the connection means and a closed position in which such passage is not possible. Preferably in the closed position, a grip member provided on the tongue frictionally engages the strap member. Preferably the strap member is held within the clip by frictional engagement only. The clip member is preferably of a plastics material.

Preferably at least part of the first and/or the second strap member is made of a fabric material. The material may include canvas or a material with similar flexibility and strength to canvas. One or more ends of the strap member may include leather or a material with similar flexibility to leather.

The strap members may each be between 10mm and 30mm in width and may be between 0.5mm and 3mm in thickness. The strap members may be between 50mm and 1.5m in length.

The measuring apparatus may comprise a bridle arrangement for fitting to a horse's head, to measure the horse's head for a bridle.

The strap may form part of a cheek piece for the bridle arrangement.

The strap may form part of a nose band for the bridle arrangement.

The strap may form part of a throat lash for the bridle arrangement.

The strap may form part of a brow band for the bridle arrangement.

The strap may form part of a rein or a pair of reins for the bridle arrangement.

The bridle arrangement may include a cheek strap including a strap as previously defined, a base of the cheek strap being formed of leather or a leather substitute for attaching to a bit. By leather substitute it is meant a material having similar properties of flexibility to leather.

The bridle arrangement may further include a head band of leather or leather substitute. A top of the cheek strap may be attached to the head band. Preferably the head band includes means for adjusting its length. The head band may include two parts connected together with connection means. The connection means may include a buckle and one of the two parts of the head band may be provided with a plurality of holes. The holes may be provided with identifying indicia, such as numbers.

Preferably the bridle arrangement further includes a brow band attached to the head band. The brow band may be fixedly attached to the head band. Preferably the brow band is provided with leather or leather substitute at its ends where it is attached to the head band, for providing a firm connection. The remainder of the brow band is preferably of a fabric material. The brow band is preferably provided with means for adjusting its length. These means may include a clip member which may be of a plastics material.

The bridle arrangement preferably further includes a throat lash including a strap as previously defined. The strap may be provided with one or more connection means.

The bridle arrangement preferably further includes a nose band. The nose band may be of "Cavesson" style including a strap for surrounding a horse's nose and a strap for passing over the horse's head. Preferably the strap for passing around the horse's nose includes leather or leather substitute where it is connected to the strap for passing over the horse's head. The strap for passing around the horse's nose preferably includes means for adjusting its length. These means may include one or more clips which may be of plastics material. The strap for passing around the horse's head preferably includes a strap as defined above.

The measuring apparatus may comprise a martingale arrangement, for fitting to a horse to measure the horse for a martingale. The martingale arrangement may comprise a running or a standing martingale.

The measuring apparatus may comprise a breast plate arrangement, for fitting to a horse to measure the horse for a breast plate.

According to the invention there is further provided a method of manufacturing a piece of equine tack, the method including the steps of: fitting a measuring apparatus as defined in any of the preceding paragraphs to a horse by adjusting the position of one or more of the strap members within the connection means; and manufacturing the piece of tack by referring to measurements taken from the measuring apparatus so adjusted.

The piece of equine tack may be a bridle.

Embodiments of the invention will be described for the purpose of illustration only with reference to the accompanying drawings in which:-
Fig. 1 is a diagrammatic perspective view of a part of a fitting bridle according to the invention;
Fig. 2 is a simplified diagrammatic perspective sketch of a part of a fitting bridle according to the invention;
Fig. 3 is a diagrammatic perspective view of a part of a fitting bridle according to the invention, fitted to a horse's head;
Fig. 4 is a diagrammatic plan view of parts making up a fitting bridle according to the invention;
Fig. 5 is a simplified diagrammatic perspective sketch of a Cavesson noseband for a fitting bridle according to the invention;
Fig. 6 is a diagrammatic plan view of parts making up a Cavesson noseband according to the invention;
Fig. 7 is a diagrammatic perspective view of the noseband of Figs. 5 and 6, fitted to a horse's head;
Fig. 8 is a diagrammatic plan view of parts making up an adjustable noseband for a fitting bridle according to the invention;
Fig. 9 is a diagrammatic plan view of parts making up a "figure 8" noseband for a fitting bridle according to the invention;
Fig. 10 is a diagrammatic plan view of a set of reins for a fitting bridle according to the invention;
Fig. 11 is a diagrammatic plan view of parts making up a breast plate fitting apparatus according to the invention;
Fig. 12 is a diagrammatic plan view of a ring attachment for the breast plate fitting apparatus of Fig. 11;
Fig. 13 is a diagrammatic plan view of a standing martingale attachment for the breast plate fitting apparatus of Fig. 11;
Fig. 14 is a diagrammatic plan view of parts making up a standing martingale fitting apparatus according to the invention;
Fig. 15 is a diagrammatic plan view of parts making up a running martingale fitting apparatus according to the invention; and
Figs. 16A and 16B are diagrammatic plan views of girth fitting apparatus according to the invention.

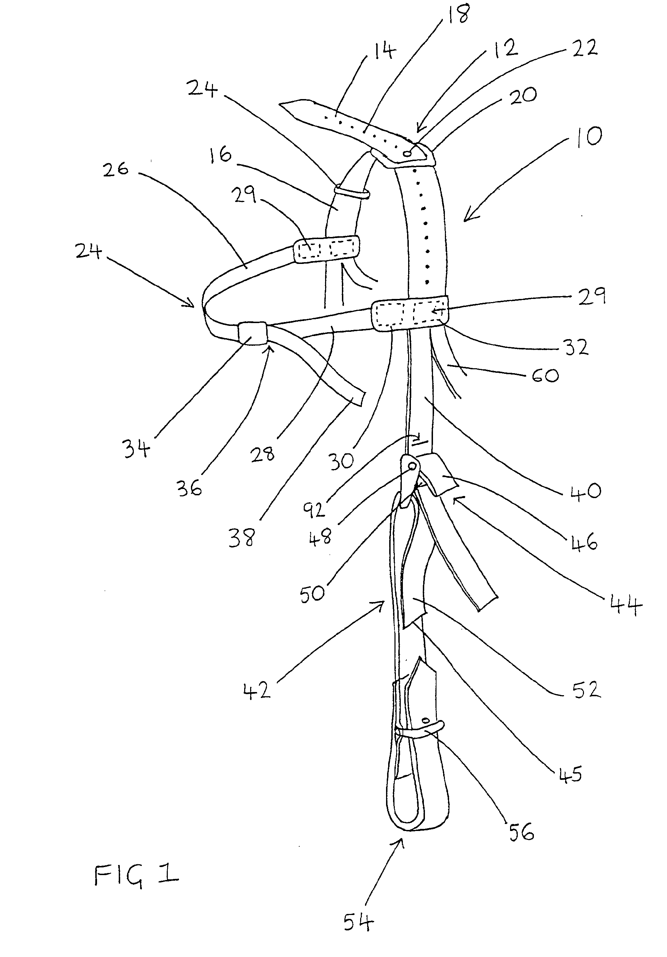

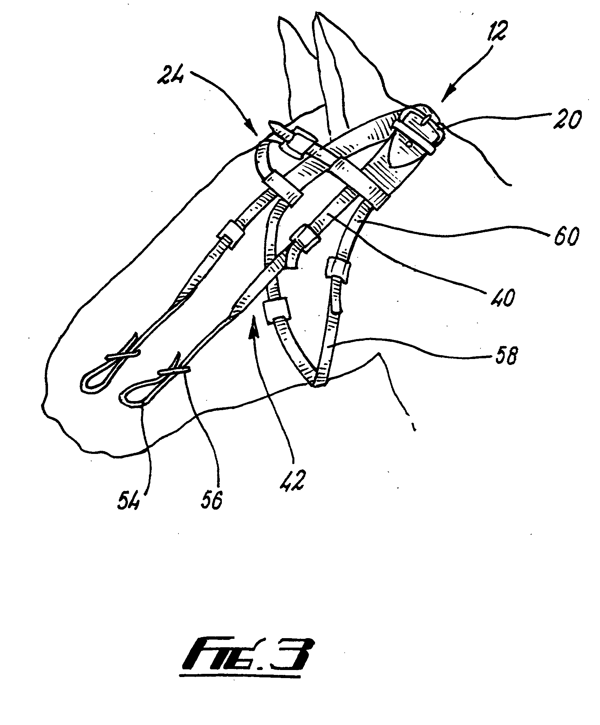

Referring to Figs. 1 to 3, there is provided a measuring apparatus in the form of a fitting bridle 10. The fitting bridle 10 includes an arrangement of straps which may be fitted on to a horse's head in order to measure the horse's head for a bridle. Fig. 1 illustrates only a part of a basic fitting bridle.

The fitting bridle 10 includes a headband 12 which is made of robust leather or a material having a similar flexibility to leather. The head piece has a width of a about 25-35mm and a thickness of about 3-5mm. The headband 12 consists of first and second strap members 14 and 16. The first strap member 14 is provided with a plurality of closely spaced holes 18 along the length thereof, and is about 250mm in length. The second strap member is about 200mm in length.

An end of the second strap member 16 is provided with a buckle 20 having a tongue 22 which may engage within one of the holes 18 in the first strap member 14. The length of the headband 12 may thus be adjusted by adjustment of the position of the first strap member 44 within the buckle 20. A keeper 24 is provided on the second strap member 16, for retaining a free end of the first strap member.

A browband 24 consists of two strap members 26 and 28 of a fabric material, such as canvas. Each strap member 26,28 includes at an end thereof an end piece 29 of leather or material of similar strength and flexibility. This leather is stitched on to the canvas material with stitching 30 and is stitched on to the leather of the headband with the stitching 32. Thus, the browband 24 is firmly and rigidly attached to the headband 12.

The first and second strap members 26,28 of the browband are connected together by a simple clip 34 of a plastics material. An end 36 of the second strap member 28 is attached to the clip 34 by being looped therethrough and stitched on to itself. An end 38 of the first strap member 26 passes through the clip 34 and when the clip 34 is in an open position the first strap member may pass freely therethrough.

The clip 34 is of conventional construction and is not illustrated in detail in the drawings. The clip includes a tongue which may be raised to allow the first strap member to pass freely through the clip or lowered to provide a frictional resistance against the first strap member 38 passing through the clip 34. In this way, the length of the browband 24 may be continuously adjusted.

A canvas strap, referred to herein as a cheek strap 40, is stitched to each end of the headband 12. Each cheek strap 40 has a width of about 15mm, a thickness of about 2-4mm and a length of about 200mm. The cheek strap 40 is attached to the headband 12 by the stitching 32.

A cheek piece 42 is connected to each cheek strap 40 via a dual clip 44. The dual clip 44 is of similar construction to the simple clip 34, but is used rather differently, and is described in more detail.

The dual clip 44 is provided with an orifice through which the cheek strap 40 may pass. When the clip is open (as described in more detail below) the cheek strap 40 may pass freely through this orifice. The clip 44 is further provided with a second orifice, located generally below the first orifice, through which an end 45 of the cheek piece 42 is passed. When the clip 44 is in its open state, the cheek strap 40 may pass freely through the clip in its respective orifice and the cheek piece 42 may also pass freely through the clip 44 in its respective orifice.

The clip 44 is provided with a tongue 46 which is able to pivot around an axle 48. On a upper, inner face of the tongue 46 there is provided a grip member 50. When the tongue 46 is pivoted in an anti-clockwise direction as illustrated in Fig. 1, the grip member 50 rotates out of engagement with the cheek strap 40 and the cheek strap 40 may thus pass freely through the clip 44. However, when the tongue 46 is rotated in the clockwise direction, the grip member engages the cheek strap 40 and holds it against a back piece (not illustrated in Fig. 1) of the clip 44. This prevents the cheek strap 40 from passing through the clip 44. In this position, the tongue 46 tends to push the cheek strap 40 against the part of a cheek piece 42 which passes through the clip 44. Thus, the cheek piece 42 is also held in place within the clip 44.

As may be seen in Fig. 1, the cheek piece 42 passes through the clip 44 and an end of the cheek piece overlaps a part of the remainder thereof. An end portion 52 of the cheek piece is provided with hook fastenings which are able to engage fleece provided along the remainder of one face of the cheek piece 42.

Thus, when the clip 44 is in its open position, the end portion 52 of the cheek piece may be pulled through the clip and folded down over the remainder of the cheek piece, thereby adjusting the length of the cheek piece 42.

A leather bit receiving member 54 is stitched on to a lower end of the cheek piece 42. The bit receiving member 54 is able to pass through a ring of a bit and buckle into position, with a buckle fastening 56. The leather bit receiving member 54 is of a similar flexibility to leather used in a conventional bridle, and thus holds the bit in the same way as would a conventional cheek piece.

It may be seen that the length of the fitting bridle between the browband 24 and the bit receiving member 54 may be adjusted by use of the clip 44. Further, the position of the clip 44 may also be adjusted. The reason for this dual adjustment is described in more detail hereinafter.

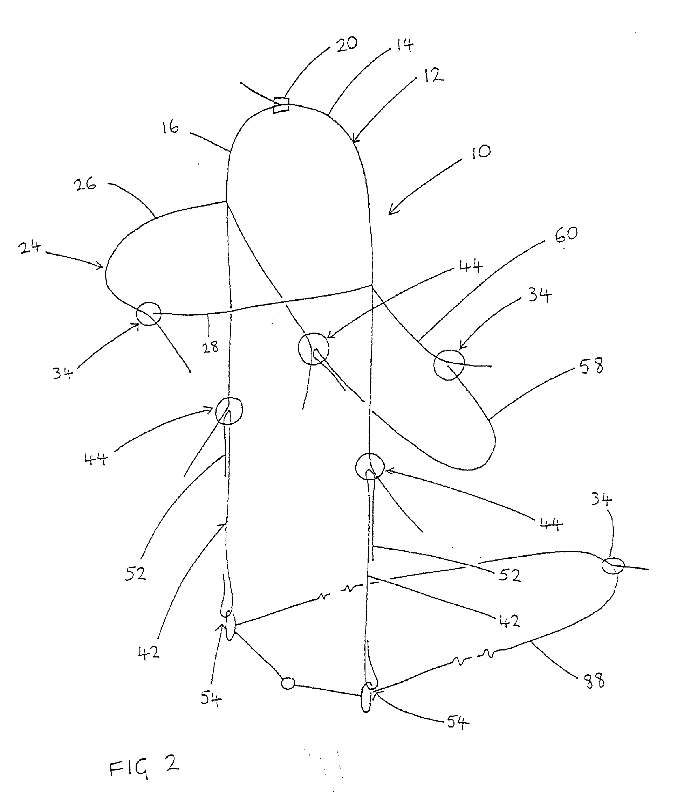

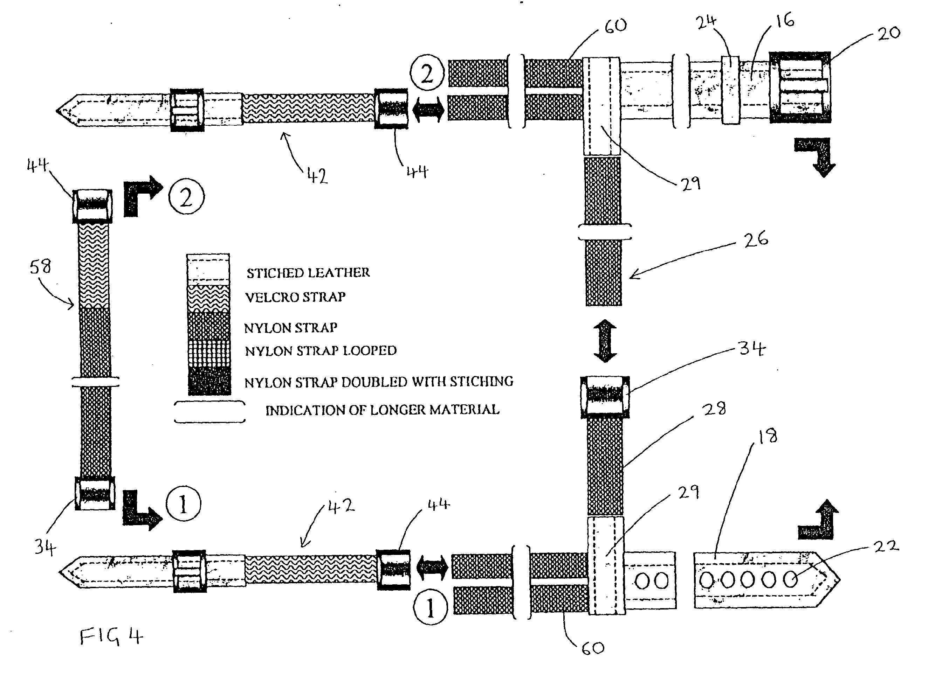

The full layout of the basic fitting bridle 10 is illustrated in Figs. 2, 3 and 4. The fitting bridle 10 includes a cheek strap 40 and a cheek piece 42 on each side of the headband 12 and the fitting bridle 10 further includes a throat lash, which is not illustrated in Fig. 1. Throat lash connector straps 60 are attached to the headband 12. The throat lash 58 is attached by clips to the throat lash connector straps 60. Referring to Fig. 2 one end of the throat lash 58 is connected to its respective throat lash connector strap 60 by a simple clip 34, while the other end of the throat lash 58 is connected to its respective throat lash connector strap by a dual clip 44. This end 62 of the throat lash connector strap is provided with hook and fleece fastenings, as described previously in relation to the cheek straps and cheek pieces, to allow its length to be altered. The clips 34 and 44 are equivalent to those provided on the browband 24 and cheek straps 40 respectively.

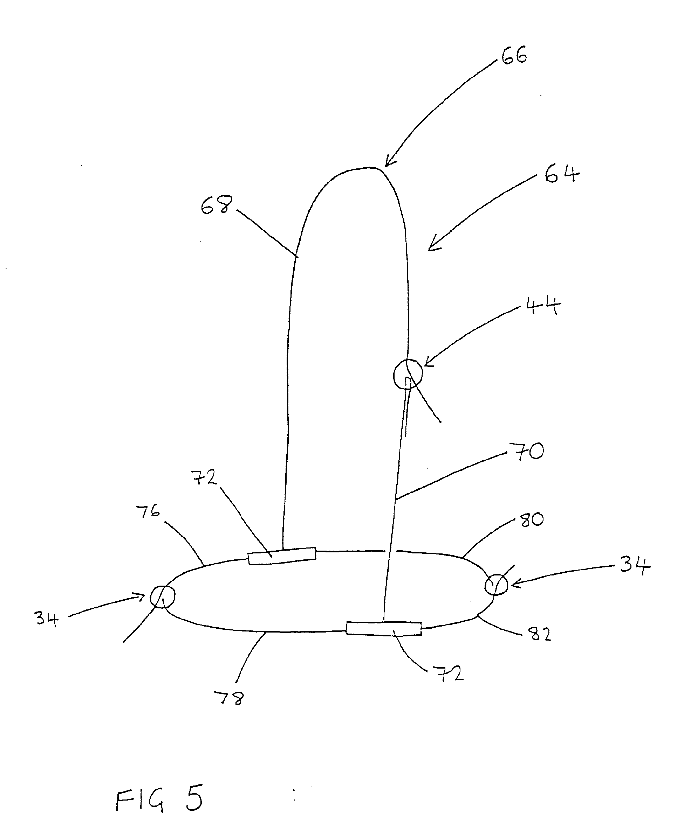

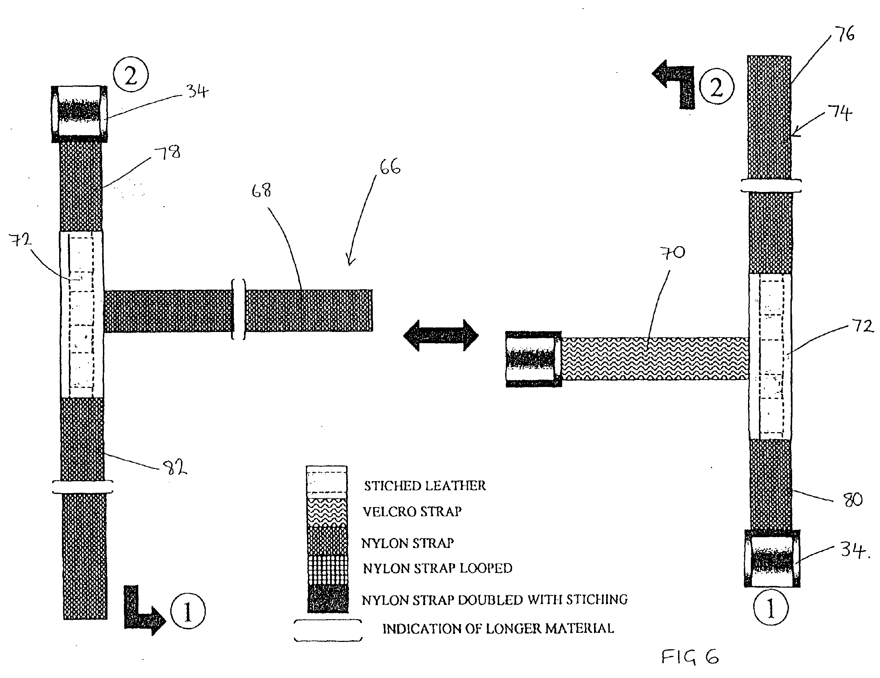

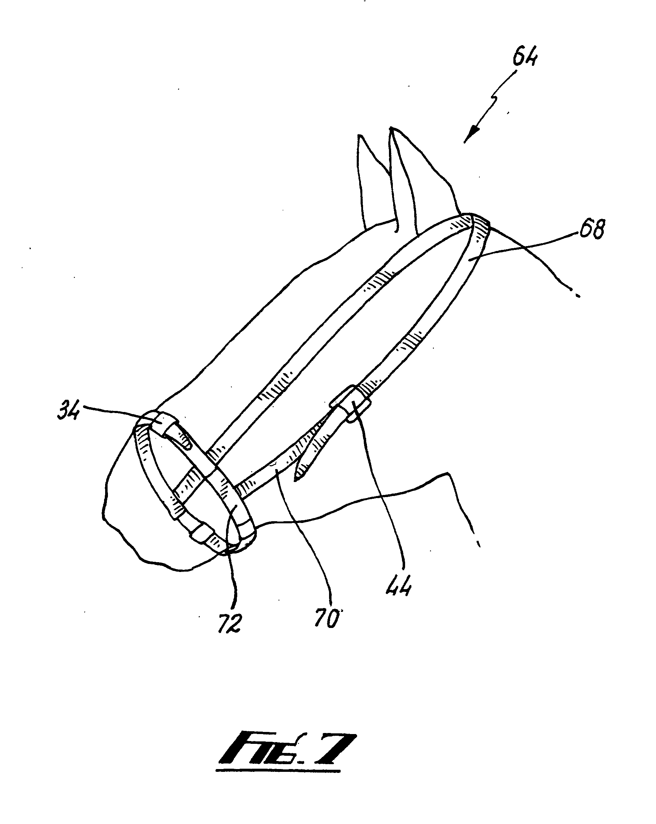

Referring to Figs. 5, 6 and 7, the fitting bridle may also include a Cavesson noseband 64. The Cavesson noseband may include a head piece 66, consisting of first and second strap members 68 and 70 connected together by a dual clip 44. A base of each strap member 68,70 may be connected by stitching to a piece of leather 72, giving a firm connection. A front part of each piece of leather 72 may be connected to a respective end of a front nose strap 74. The front nose strap 74 includes first and second strap members 76 and 78 connected together by a simple clip 34. The backs of the pieces of leather 72 are connected to respective ends of a back nose strap 78. The back nose strap 78 is made up of two strap members 80 and 82, connected together by a simple clip 34.

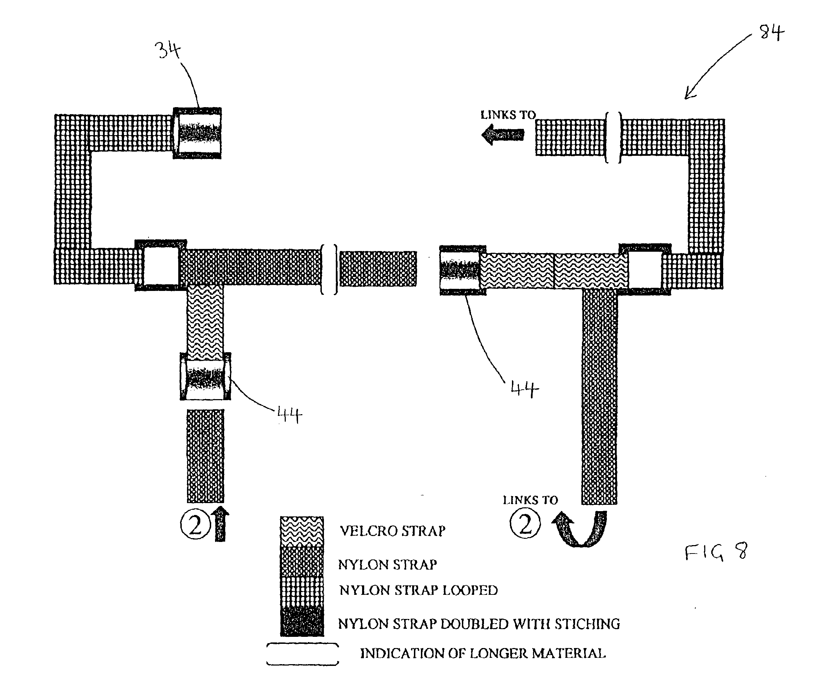

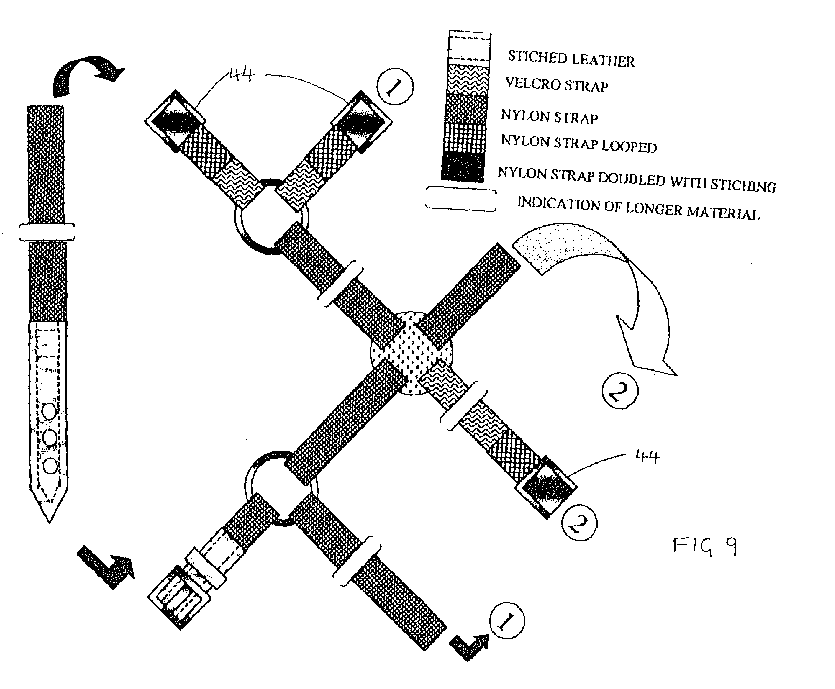

Instead of a Cavesson noseband 64, the fitting bridle 10 may be provided with an adjustable noseband 84, as illustrated in Fig. 8 or a "Figure 8" noseband as illustrated in Fig. 9. In each case, the noseband includes straps connected together with simple and/or dual clips 34 and 44 respectively, thus ensuring full adjustability.

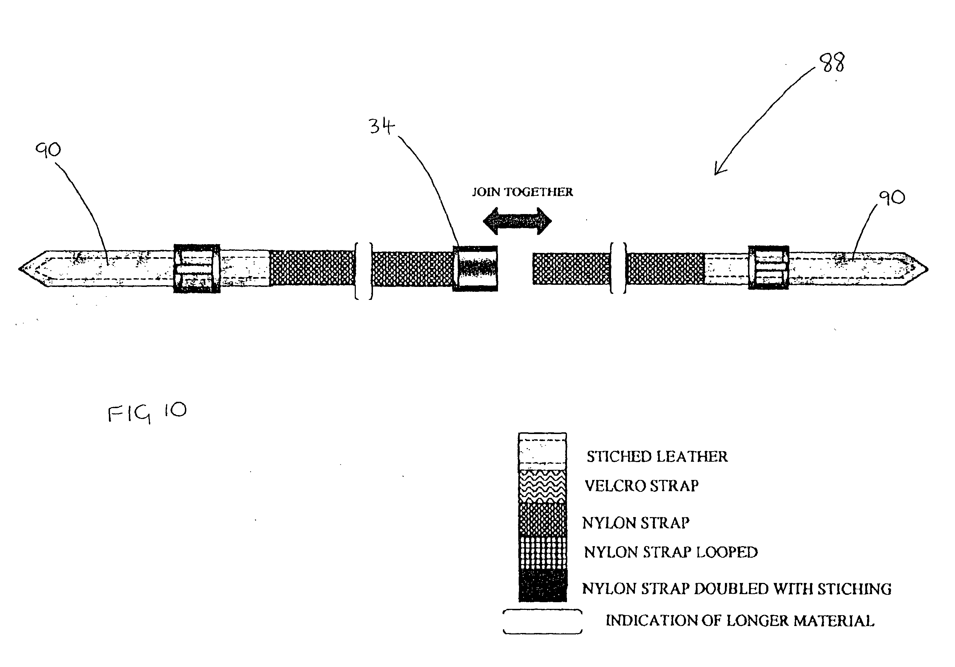

Referring to Fig. 10, the fitting bridle 10 is further provided with reins 88 provided with leather bit connecting ends 90 and a simple clip 34.

The fitting bridle 10 is used as follows. A horse owner adjusts the clips 34 and 44 on the fitting bridle 10 so that the fitting bridle is of approximately the right overall size for the horse. The fitting bridle 10 may be adjusted such that it is a generally similar size to the horse's existing bridle, particularly in the distance between the two buckle fastenings 56, over the headband 12. The horse owner's existing bit is also fitted to the bit receiving members 54. The fitting bridle 10 may then be put on to the horse. The headband 12 is adjusted, by altering the position of the first strap member 18 within the buckle 20, so that the browband 24 is positioning correctly on the horse's head. The browband should not be too high and tight on the horse's ears or too low, near the horse's eyes.

The clips 44 connecting the cheek straps 40 and the cheek pieces 42 may be adjusted so that the bit lies correctly in the horse's mouth. Because the bit, receiving members 54 are made of bridle leather, the bit should lie at the same height within these members as it would within a finished bridle. The clips 44 may be adjusted in two ways: by varying the extent of overlap of the end portion 52 of the cheek piece 42 or by drawing the cheek strap 40 through the clip 44. Thus, not only may the length between the bit receiving member 54 and the browband 24 be altered, also the precise positioning of the clip 44 on the horse's face may be adjusted. Thus, it may be ensured that the clip 44 does not lie uncomfortably on any of the bones in the horse's face. The position of the clip represents the position of a buckle in the eventual bridle to be made using the fitting bridle. One or more guide indicia 92 (see Fig. 1) may be provided on the cheek straps 40, to give a general indication of a suitable location for the clip 44.

The length of the browband 24 may be altered using the clip 34, and the length of the throat lash may be adjusted using its two clips 34 and 44. The dual adjustment of the clip 44 allows the position of the eventual buckle or buckles in the throat lash to be chosen.

The noseband 64 may be adjusted in a similar way, to ensure a perfect fit at the front and the back of the horse's nose and to make sure that the noseband again lies comfortably on the bones in the horse's face. The reins 88 may also be adjusted to give a perfect fit.

Once the horse owner is happy with the fit of the fitting bridle 10 on their horse's head, they note the number of the hole 18 in the headband 12 in which the tongue 22 of the buckle 20 lies. This buckle 22 is then undone and the fitting bridle removed without undoing any of the other clips. The fitting bridle 10 may then be returned to the bridle manufacturer, who uses it to make a made-to-measure bridle. In this way, a bridle may be made which fits the horse perfectly.

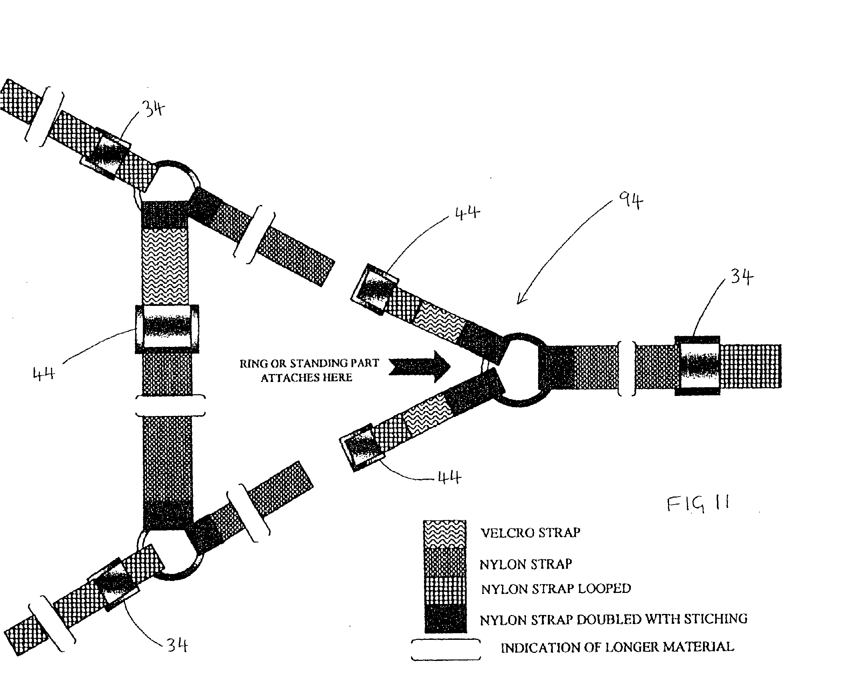

The invention also provides measuring apparatus for additional tack, as illustrated in Figs. 11 to 16. Fig. 11 illustrates a breast plate fitting apparatus 94, which is made up of straps and clips 34 and 44 which function in a similar manner to that described in relation to the fitting bridle 10. The breast plate fitting apparatus 94 may be adjusted and returned to the bridle maker for making up into a breast plate which fits perfectly.

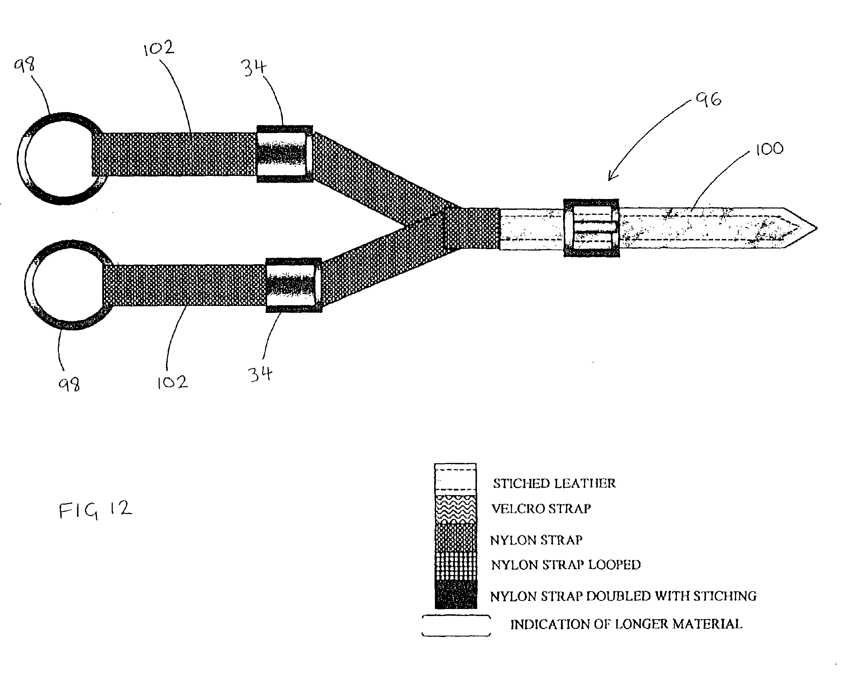

Fig. 12 illustrates a running martingale attachment 96 for a breast plate, the running martingale attachment 96 including loops 98 for receiving the reins, and a leather strap 100 for attachment to a breast plate. Straps 102, provided with simple clips 34, link the loops 98 to the strap 100.

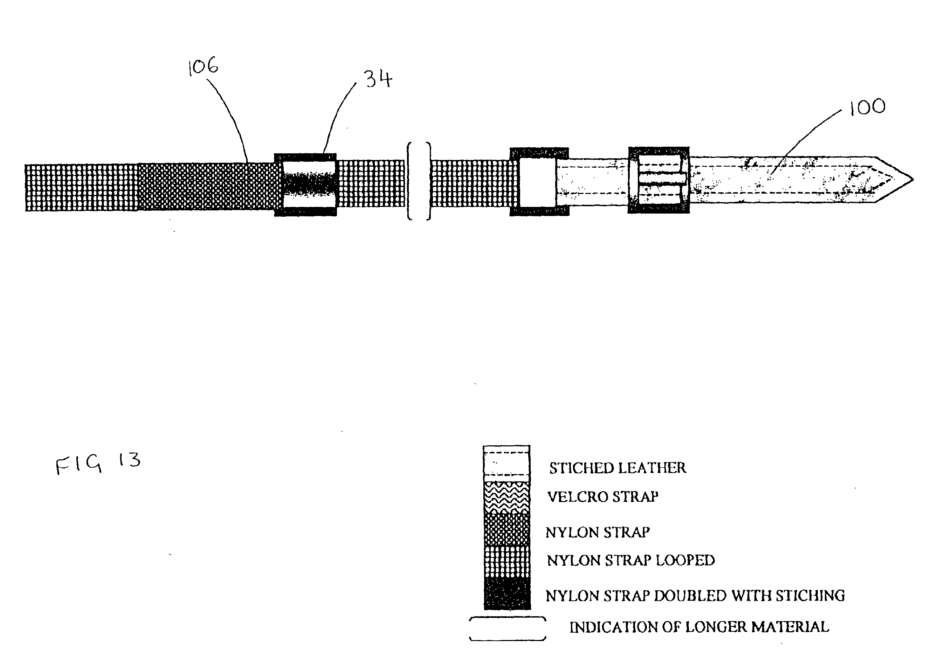

Fig. 13 illustrates a standing martingale attachment 104 for a breast plate. The standing martingale attachment 104 includes an adjustable strap 106, provided with a simple clip 34, for attaching to the noseband of a bridle. The standing martingale attachment further includes a leather strap 100 for attachment to the breast plate.

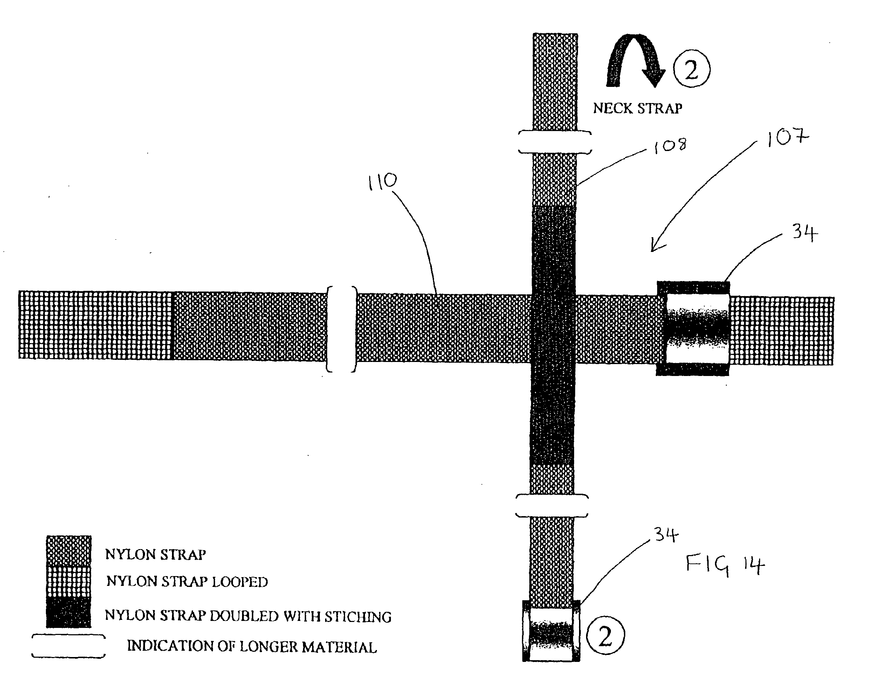

Fig. 14 illustrates a standing martingale fitting apparatus 107, including a neck strap 108, provided with a simple clip 34. The standing martingale fitting apparatus 107 further includes an adjustable strap 110 for attachment to a noseband of a bridle and to a girth of a saddle, in the usual way. The strap 110 is provided with a simple clip 34.

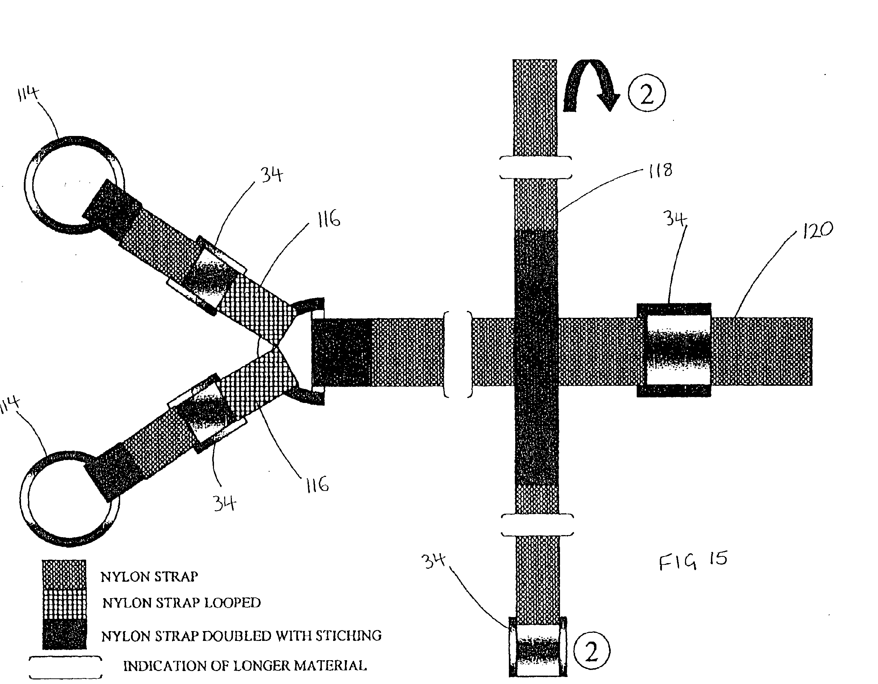

Fig. 15 illustrates a running martingale fitting apparatus 112. The running martingale fitting apparatus is provided with loops 114 for receiving reins, the loops being provided at the ends of adjustable straps 116, provided with simple clips 34. A neck strap 118 is also provided with a simple clip for adjustment, as is a strap 120 for attachment to a girth of a saddle.

All the above measuring apparatus may be used to measure up a horse for the appropriate piece of tack.

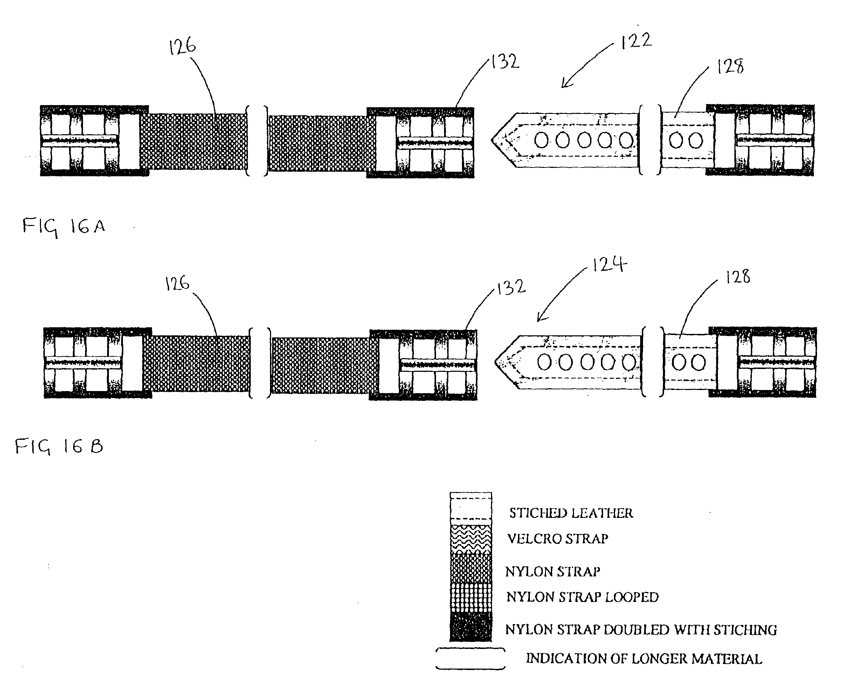

Finally, Figs. 16A and 16B illustrate a short dressage girth fitting apparatus 112 and a long standard girth fitting apparatus 124 respectively. Each girth fitting apparatus includes a nylon strap 126 and a stitched leather portion 128, provided with a plurality of numbered holes 130. The dressage girth fitting apparatus 122 includes seven holes and the standard girth fitting apparatus 124 fifteen holes. A buckle 132 connects the nylon strap 126 and the leather portion 128 in each case. Each girth fitting apparatus may be adjusted to provide a perfect fit and sent back to the bridle maker so that the bridle maker may make a made-to-measure girth for the horse.

There is thus provided measuring apparatus which allows a bridle maker to make a made-to-measure bridle or other tack for a horse, without having to physically measure the horse.

Various modifications may be made to the above described embodiment without departing from the scope of the invention. The materials used may be modified, and alternative clips may be used for example metal clips including a tongue member spring biased into a position where it grips one or more straps of the bridle arrangement. However, the clips and materials are preferably clearly unsuitable for riding in.

## Claims

1. Measuring apparatus (10) for use in making equine tack, the measuring apparatus including a strap of adjustable length, the strap being made up of a first strap member (40) and a second strap member (42) joined by connection means (44), whereby the position of the first strap member (40) within the connection means may be adjusted to thereby adjust the length of the strap, and wherein the connection means (44) is alterable between a state in which it allows the first strap member (40) to slide therethrough and a state in which such sliding is not possible.

2. Measuring apparatus according to claim 1, wherein the second strap member (42) passes through the connection means (44) and partially overlaps itself such that the length of the strap may be adjusted by varying the extent of this overlap.

3. Measuring apparatus according to claim 2, wherein the extent of overlap is continuously variable.

4. Measuring apparatus according to claim 2 or claim 3, wherein the overlapped portion of the second strap member (42) may be attached to the remainder of the second strap member by a hook and fleece fastening.

5. Measuring apparatus according to claim 4, wherein an end portion (52) of the second strap member is provided with hooks and the remainder of the strap member with fleece.

6. Measuring apparatus according to any preceding claim, wherein the position of the first strap member (40) within the connection means is adjustable.

7. Measuring apparatus according to claim 6, wherein the first strap member (40) is provided with one or more indicia for indicating a preferred position or positions within the connection means.

8. Measuring apparatus according to any preceding claim, wherein when the connection means (44) is in the open state, the second strap member (42) is also be able to slide therethrough.

9. Measuring apparatus according to any preceding claim, wherein the connection means (44) includes a clip member provided with a tongue (46) which is pivotable between an open position in which the first and/or second strap member may pass through the connection means and a closed position in which such passage is not possible.

10. Measuring apparatus according to claim 9, wherein in the closed position, a grip member (50) provided on the tongue frictionally engages the strap member.

11. Measuring apparatus according to claim 10, wherein the strap member is held within the clip by frictional engagement only.

12. Measuring apparatus according to any preceding claim, wherein at least part of the first (40) and/or the second (42) strap member is made of a fabric material.

13. Measuring apparatus according to any preceding claim, wherein the measuring apparatus comprises a bridle arrangement for fitting to a horse's head, to measure the horse's head for a bridle.

14. Measuring apparatus according to claim 13, wherein the strap forms part of a cheek piece, a nose band, a throat lash, a brow band and/or a rein or pair of reins for the bridle arrangement.

15. Measuring apparatus according to claim 14, wherein the bridle arrangement includes a cheek strap including a strap as previously defined, a base of the cheek strap being formed of leather or a leather substitute for attaching to a bit.

16. Measuring apparatus according to claim 15, the bridle arrangement further including a head band (12) of leather or leather substitute and wherein a top of the cheek strap is attached to the head band.

17. Measuring apparatus according to claim 16, wherein the head band (12) includes two parts (14,16) connected together with connection means (20), one of the two parts of the head band being provided with a plurality of holes (18) having identifying indicia.

18. Measuring apparatus according to claim 16 or claim 17, wherein the bridle arrangement further includes a brow band (24) fixedly attached to the head band.

19. Measuring apparatus according to claim 18, wherein the brow band (24) is provided with leather or leather substitute at its ends where it is attached to the head band, for providing a firm connection, and wherein the remainder of the brow band is of a fabric material.

20. Measuring apparatus according to claim 19, wherein the brow band is provided with means for adjusting its length.

21. Measuring apparatus according to any of claims 13 to 20, wherein the bridle arrangement further includes a throat lash (58) including the strap.

22. Measuring apparatus according to any of claims 13 to 21, wherein the bridle arrangement further includes a nose band (64) including a strap (74,78) for surrounding a horse's nose and a strap (66) for passing over the horse's head, wherein the strap for passing around the horse's nose includes leather or leather substitute where it is connected to the strap for passing over the horse's head and the strap for passing around the horse's nose includes means for adjusting its length.

23. Apparatus according to any of claims 1 to 12, wherein the measuring apparatus comprises a martingale arrangement (96,104,107), for fitting to a horse to measure the horse for a martingale.

24. Apparatus according to any of claims 1 to 12, wherein the measuring apparatus comprises a breast plate (94) arrangement, for fitting to a horse to measure the horse for a breast plate.

25. A method of manufacturing a piece of equine tack, the method including the steps of: fitting a measuring apparatus as defined in any of the preceding claims to a horse by adjusting the position of one or more of the strap members within the connection means; and manufacturing the piece of tack by referring to measurements taken from the measuring apparatus so adjusted.

26. A method according to claim 25, wherein the piece of equine tack is a bridle.
